# EUROPEAN PATENT SPECIFICATION

(11) **EP 2 668 952 B1**
(45) Date of publication and mention of the grant of the patent: **08.02.2017**
(21) Application number: 13159132.3
(22) Date of filing: 14.03.2013
(51) Int. Cl.: A61K 31/592, A61K 31/593, A61K 31/663, A61K 47/10, A61K 9/00

(54) **Aqueous formulations of bisphosphonates, vitamin D and benzyl alcohol suitable for subcutaneous or intramuscular use**
Wässrige Formulierungen aus Bisphosphonaten, Vitamin D und Benzylalkohol, die zur subkutanen oder intramuskulären Verwendung geeignet sind
Formulations aqueuses de bisphosphonates, de vitamine D et d'alcool benzylique appropriées pour une utilisation sous-cutanée ou intramusculaire

(30) Priority: 14.03.2012 IT MI20120393
(43) Date of publication of application: 04.12.2013
(73) Proprietor: Romano, Paolo, 20146 Milano (IT); Bruzzese, Tiberio, 20146 Milan (IT)
(72) Inventor: Romano, Paolo, 20146 Milano (IT); Bruzzese, Tiberio, 20146 Milan (IT)
(74) Representative: Ferreccio, Rinaldo

(56) References cited:
- WO-A1-2005/044280
- WO-A1-2008/116133
- WO-A1-2008/116809
- WO-A1-2011/063952
- GB-A- 2 414 181
- KR-A- 20110 030 852
- KIBBE A H: "BENZYL ALCOHOL", 1 January 2000 (2000-01-01), HANDBOOK OF PHARMACEUTICAL EXCIPIENTS, ED.3, AMERICAN PHARMACEUTICAL ASSOCIATION ET AL, USA, PAGE(S) 41,191 - 220,,372,442, XP002533446, * page 1, paragraph 7 *

## Description

This invention relates to compositions for pharmaceutical use which comprise a compound belonging to the class of bisphosphonates (BFs) dissolved in an aqueous solution, along with vitamin D and benzyl alcohol, in volumes that are suitable for intramuscular or subcutaneous administration.

Bisphosphonates are a class of drugs that suppress bone metabolism by inhibiting the action of osteoclasts (RG Russell et al. J Bone Miner Res 1999; (Suppl) 2:53) and hence are used effectively in many diseases with increased bone turnover, both of metabolic type (eg. osteoporosis and Paget's disease) and of tumor origin (eg. bone metastases).

In this use, bisphosphonates are administered either orally or parenterally (e.g., intravenously and intramuscularly), with daily (when orally administered) or periodic (weekly, monthly, quarterly or yearly) in the case of oral or parenteral schedules of administration (Cremers et al. Clin Pharmacokinet. 2005; 44:551).

Since the percentages of absorption from the oral route are extremely low, from 0.7% to about 2%, depending on the type of bisphosphonate and the administered dose (Cremers S and Papapoulos S. Bone. 2011; 49:42) and with a considerable inter-individual variability, the parenteral route, although more invasive, appears the most rational mode of administration of these drugs, also in consideration of the possibility to adopt periodic schemes of therapy. Among the parenteral options, the intramuscular and subcutaneous ones are the most practical, being easily performed at home without resorting to specialized staff or to health care facilities, as in the case of the intravenous one.

The periodic parenteral administration, i.e. weekly or at longer intervals of time, however, involves the administration of high doses of bisphosphonates with a great osteo-inhibiting power which are rapidly absorbed into the body. This exposes the patient to the risk of hypocalcemia, which will be the greater, the lower the levels of calcium in the blood are (Hananamura M et al. Biol Pharm Bull. 2010; 33:721).

Vitamin D (cholecalciferol and ergocalciferol) is synthesized in the skin following exposure to sunlight or taken with the diet. In its most active forms, i.e. after hydroxylation at position 25 (in the liver) and at position 1 (in the kidney), respectively, to form 25-hydroxy-cholecalciferol and 1,25-dihydroxy-cholecalciferol, vitamin D plays a very important role in the regulation of bone metabolism and calcium phosphate homeostasis. In general, optimal levels of vitamin D facilitate the absorption of calcium and phosphorus by the kidney and intestine (DeLuca HF. Am J Clin Nutr. 2004; 80 (Suppl): 1689S-96S) and the maintenance of correct plasma levels of these elements while lower levels cause the resorption of bone tissue (which acts as a deposit of the same).

Lack of vitamin D on the other hand is a very common condition in every part of the world and in various kind of subjects (Thomas MK. Et al. N Engl J Med 2008; 338:777; 357:266-281; Holick MF . N Engl J Med 2007; 357:266).

These functions of vitamin D and its frequent deficiency in the population endorse the importance of its administration in conjunction with the administration of bisphosphonates, for both metabolic and oncologic indications. In support of this, the summary of product characteristics of all latest bisphosphonates reports the recommendation of a concomitant treatment with vitamin D, orally or intramuscularly, at variable doses and also with a loading dose.

In the course of treatment with bisphosphonates, therefore, supplementation with vitamin D is considered very useful both to avoid possible episodes of hypocalcemia, particularly as a result of high doses of parenteral bisphosphonates, and to improve the therapeutic response to bisphosphonates (Tanvetyanon T and Stiff PJ. Ann Oncol. 2006; 17:897).

Since, in general, the compliance to treatments, including those for osteoporosis (Cramer JA et al. Osteoporosis Int 2007; 18:1023), is often not quite high, the possibility of administering a bisphosphonate and vitamin D in a fixed combination appears useful to facilitate the correction or prevention of hypovitaminosis, thus increasing the efficacy of bisphosphonates therapy and reducing the risk of their side effects.

To demonstrate this utility, there is also the example of the introduction on the market of a fixed combination of a bisphosphonate (alendronate sodium) with vitamin D, for oral administration (SH Ralston et al. Calcif Tissue Int 2011; 88:485).

Until now, however, similar combinations of injectable preparations for intramuscular or subcutaneous administration are not commercially available due to the different solubility of the two components: the bisphosphonates are in fact water-soluble compounds, while vitamin D is fat-soluble.

In truth, we are aware of the existence of two patent applications (WO 2008/116809 and WO 2011/063952) claiming the use in combination of bisphosphonates and vitamin D, parenterally.

However, the first of these (WO 2008/116809) is related to formulations which, because of their high volume (100 ml in all the examples, to be administered as an infusion over at least 15 minutes), are unsuitable for administration by intramuscular or subcutaneous route. Furthermore it is observed that such formulations contain settling particulate material when polysorbate is used as a dispersant (examples 1-7), or have an opalescent appearance when for this purpose lecithin is used (examples 13-17).

The second of the above-mentioned patent applications (WO 2011/063 952), through the use of a higher concentration of lipids and phospholipids in water, has allowed to overcome the problem of the solubility of vitamin D and bisphosphonates at high concentrations and in small volumes, so as to be suitable for intramuscular or subcutaneous intermittent administration, also at quite long intervals. The adopted solution also allows to improve the intramuscular or subcutaneous absorption of vitamin D, compared to formulations with purely oily vehicles, and to optimize the kinetics of absorption of bisphosphonates by slowing it down.

However, the physical and chemical stability of these formulations (bisphosphonates and vitamin D, vehicled in lipid emulsion) is not optimal, since the molecules of bisphosphonates tend to cause separation of the oily phase of the emulsion, especially when exposed to high temperatures as in the process of steam sterilization. At the same time, the sterilization of an emulsion containing a bisphosphonate and vitamin D through sterilizing filters is also not easy because of the size of the fat particles of the usual emulsions (often > 0.22 µm); for this reason it may be necessary to follow production processes which allow to obtain an emulsion particles with more homogeneous and smaller diameter (also through a pre-filtration) or microemulsions (with lipid particles less than 0.1 microns) although this may require the use of higher emulsifier concentrations which may be excessive for clinical use.

We have found conversely not only that the use of benzyl alcohol as a solvent of vitamin D allows to dissolve very well the vitamin D itself, but also, and in particular, that vitamin D remains optimally solubilized even when one subsequently carries out dilution in water for injections, to volumes suitable for administration by intramuscular or subcutaneous route and this even in the presence of the bisphosphonate at the concentrations useful for the correction of abnormalities of bone metabolism. In fact, these final preparations containing benzyl alcohol, vitamin D and a bisphosphonate in aqueous solution, do not show any sign of solid precipitation and appear perfectly clear, without particulate material suspended. Likewise, vitamin D can be effectively dissolved even in an aqueous solution containing benzyl alcohol already diluted to the final desired values.

According to one aspect, the present invention thus relates to a composition for pharmaceutical use according to claim 1. Preferred embodiments of such a composition are reflected by dependent claims 2 to 10.

According to another aspect, the present invention relates to a pharmaceutical formulation for intramuscular and subcutaneous administration according to claim 11, including the above composition. Preferred embodiments of such a pharmaceutical formulation are detailed in claims 12, 13 and 17-18. A method for producing the pharmaceutical formulation of the invention is detailed in claims 14-16.

Compared to formulations that use lipid emulsion, at a concentration between 5% and 40%, to solubilize vitamin D, which require a pH toward alkalinity for a better stability of the emulsion itself, the formulation according to the present invention shows the advantage of displaying, at identical conditions of temperature, exposure to light and other variables, a reduced degradation of vitamin D over time. Furthermore with respect to formulations using the lipid emulsion as a vehicle of vitamin D and bisphosphonates, the formulation according to the present invention can easily undergo a final moist heat sterilization (eg 121°C for 15-30 minutes) without any impairment of the properties of the formulation; alternatively, unlike preparations in lipid emulsion, the formulation according to the present invention can easily undergo a sterile filtration process through filters with pores of 0.22 µm or less.

More particularly, the composition of the present invention comprises a bisphosphonate in an amount between 400 mg and 1 mg, preferably between 200 mg and 3 mg, in which the highest value can be represented by clodronic acid, at a suitable dose for administration every 1-2 weeks and the lowest one by ibandronic acid, suitable for administration every 3 months, and vitamin D in a dose variable between 2700 IU to 900,000 IU as required by administrations at intervals of 1 week (at the lowest accepted doses) or one year (at the highest dosages among those accepted), and preferably in a dosage even more restricted between 5600 IU and 90,000 IU, suited to single dosing in periods between 1 week (at highest dosages among those accepted) and 3 months.

The concentration of benzyl alcohol in the formulation may vary between 0.3% (3 mg/ml) and 10% (100 mg/ml) and preferably in a still narrower range of 0.5% (5 mg/ml) to 5% (50 mg/ml) and even more preferably between 1% (10 mg/ml) and 3% (30 mg/ml), while the final volume of the formulation will be between 0.5 and 5.0 ml and preferably between 0.5 and 3.5 ml, and still more preferably between 1 and 2.0 ml; it follows that the concentration of vitamin D will never be less than 540 IU/ml or more than 1.8 million IU/ml.

Even more surprising is that vitamin D solubilized in such preparations with benzyl alcohol (and also containing a bisphosphonate), as confirmed by experimental comparative tests in the animal, is absorbed from the injection site more rapidly than in the case of formulations where vitamin D is solubilized with polysorbate or lecithin or with lipid emulsions, reaching at the same observation time, higher plasma concentrations. Finally, we must add that benzyl alcohol is able to improve local tolerability of intramuscular or subcutaneous administration for its known anti-nociceptive properties (Poli G. et al. Eur J Drug Metab Pharmacokinet. 2004; 29:145).

Moving on to a more detailed examination of the composition of the invention, this comprises at least one of the BFs known in the literature such as clodronate, alendronate, etidronate, neridronate, pamidronate, risedronate, zoledronate, ibandronate, incadronate, olpadronate, tiludronate, alone or in combination, all usable and available in the various possible forms (acids or salts with bases acceptable from the pharmaceutical point of view, typically the sodium salts, all at various degrees of salification; anhydrous or hydrated forms; the racemates, the enantiomers and diastereoisomers, if eligible, and the various crystalline forms, amorphous and polymorphic).

The dose of the BFs will depend on their specific power and, compared to the oral dose, should reflect the proportion of bioavailable drug, that is around 1% of the oral dose whereas in the intramuscular or subcutaneous formulation the absorption will settle around 100%.

The dose of BF then will depend, of course, also on the frequency of the intermittent administration envisaged in the present invention, indicatively 7, 30, 90 times the daily dose, respectively for weekly, monthly or quarterly administration, and so on.

Typical doses will be indicatively of 100 mg or 200 mg for the clodronate (every 1-2, up to 2-4 weeks); 25 mg or 50 mg for the neridronate (every 2 - 4 weeks); 1.5 mg, 2, 5 mg, 5 mg of zoledronic acid (every 3, 6, 12 months). In general terms, the content of BF will be between 400 mg and 1 mg, preferably between 200 mg and 3 mg.

Vitamin D is mainly represented by cholecalciferol (vitamin D3), in a dose within the range between a minimum of 2700 international unit (IU) up gradually to 900,000 IU for weekly, monthly or annual administration, in an average unit dose containing at least 2700 IU, at least 20,000 IU and at least 60,000 IU to be administered once every week, every month and every quarter and, preferably, 5600 IU, 30,000 IU and 90,000 IU, respectively, every week, month and quarter.

Another vitamin of the D group that can be used is ergocalciferol (vitamin D2), at the same doses of vitamin D3

All of these compositions of the invention based on BF and vitamin D have a volume strictly between 0.5 and 5.0 ml and preferably between 0.5 and 3.5 ml, and still more preferably between 1 and 2.0 ml, and are exclusively reserved for intramuscular and subcutaneous administration as injectable bolus. The slow infusion is totally impractical and is still meaningless in clinical terms. As injectables, the compositions will necessarily be sterile and equipped with the other properties listed hereunder.

The compositions of formulations for pharmaceutical use also include one or more excipients and/or co-adjuvants acceptable from the pharmaceutical point of view, as known to the expert for the purposes of an injectable formulation, which, also in this case, must provide for a total solubilization of the active ingredients, an optimal pH value, an isotonic solution, etc..

These substances may belong to the group of sugars, such as glucose, sucrose, lactose or mannose; to the group of polyhydric alcohol, such as glycerol, xylitol and others; preservatives such as parabens; of ionic and non-ionic surfactants, such as sodium deoxycholate, sodium glycocholate and other bile salts, sodium lauryl sulfate, of Tween and Cremophor; of basic, neutral or acidic buffer, such as alkali carbonates or bicarbonates, phosphates or TRIS buffer or in general to the group of organic solvents miscible in water and pharmaceutically acceptable, in small quantities.

The use of basic (such as sodium bicarbonate) or acidic (such as phosphoric acid) buffer, is particularly suitable to bring the pH of the emulsion to values compatible with the intramuscular and subcutaneous administration, such as between 3 and 9, or most preferably between 5 and 7, thus neutralizing the strong acidity of the solutions of BF and also contributing to a better tolerability at the site of injection.

Also frequent can be the addition of the usual local anesthetics, typically lidocaine, for the same purpose of a better local tolerability.

The composition usually also contains antioxidants, such as tocopherol, ascorbyl palmitate, butyl-hydroxyanisole and butyl-hydroxytoluene; and also stabilizers, co-solvents substances, etc ..., as known to the expert. Likewise, the composition of the formulations can be suitably protected in an inert atmosphere such as nitrogen.

The composition of the invention, in formulation for pharmaceutical use, can be produced and presented in mutually different ways.

According to a first method, which is also preferred, the composition contains directly the desired BF and vitamin D, dissolved in aqueous solution together with benzyl alcohol and any other possible excipient and/or adjuvant, and is sterile, ready for administration, as a single composition, of the two active ingredients. As mentioned above, the composition is dedicated to the exclusive intramuscular and subcutaneous use in bolus, in doses that are multiples of those suitable for a theoretical daily use and therefore for a highly intermittent administration.

According to a further method, the BF is in aqueous solution, together with any excipients and/or adjuvants, while vitamin D is dissolved in benzyl alcohol together with possible excipients and/or adjuvants, and then the two separate solutions, in a suitable volume and concentration are brought together extemporaneously before administration and then injected, or are injected separately in a sequential manner. In a variant of said method, the BF and the vitamin D are present in a lyophilized form, obtained according to the methods known to the expert, and are dissolved in an aqueous solution containing benzyl alcohol, prior to administration and then injected. Alternatively, vitamin D instead than in the lyophilized preparation may be present in the diluent solution together with benzyl alcohol.

No difficulty is related to the dissolution of the BF in water, which represents the preferred procedure; equally practical is the direct dissolution of the BF in aqueous benzyl alcohol, preferably - but not necessarily - already containing the vitamin D dissolved.

Regarding vitamin D, this could also be dissolved in the aqueous solution already containing benzyl alcohol, in which the BF is possibly also already dissolved, or it is dissolved in benzyl alcohol and the solution is subsequently diluted with water for injections. Controls include of course the other aspects related to the stability of the active substances and excipients, the isotonicity and sterility of the compositions. Extemporaneous preparations are easily carried in prefilled syringes.

The sterility of the injectable formulations according to the present invention may be obtained in various ways, such as for example final moist heat sterilization or sterile production followed by sterile filtration.

The following examples are intended to further illustrate the compositions of the invention and their preparation method, without, however, having any restrictive intent. Other examples are intended to highlight the unexpected biological results, obtained in animals studies carried out in laboratories, related to the effect of benzyl alcohol on the absorption of vitamin D.

### Example 1

Composition:

| | |
|---|---|
| Disodium clodronate | 200 mg |
| Cholecalciferol (14,000 IU) | 0.35 mg |
| Benzyl alcohol | 70 mg |
| Sodium bicarbonate | q.s. to pH 7 |
| Water for injections, | q.s. to 3 ml |

Vitamin D is dissolved in benzyl alcohol; subsequently it is diluted with water for injections and the sodium clodronate is added together with sodium bicarbonate (alternatively the clodronate can be previously diluted in water); it is then shaked and distributed in vials or neutral glass ampoules, and moist heat sterilized in autoclave (121°C for 15 minutes). Alternatively, sterility can be achieved by filtration through a 0.22 µm filter before the partition in vials or ampouls. It is administered intramuscularly, for example at the rate of one injection every 2 weeks.

### Example 2

Composition:

| | |
|---|---|
| neridronate sodium | 25 mg |
| Water for injections, | q.s. to 2 ml |
| Cholecalciferol (14,000 IU) | 0.35 mg |
| Benzyl alcohol | 35 mg |
| Buffering agent | q.s. at pH 6.5 |

The preparation is carried out as in Example 1., by replacing the air with a mixture of nitrogen, as known to the expert; the preparation is suitable for intramuscular or subcutaneous administration every 14 days.

### Example 3

Composition:

| Vial A | |
|---|---|
| Ibandronic acid | 3 mg |
| Buffering agent | q.s. at pH 7 |
| Mannitol | 325 mg |
| Water for injections, | q.s. to 3 ml |

| Vial B | |
|---|---|
| Benzyl alcohol | 35 mg |
| Cholecalciferol (90,000 IU) | 2.1 mg |
| Water for injections | q.s. to 1 ml |

Preparation: in vial A, a mixture of ibandronic acid, mannitol, buffering agent, dissolved in an aqueous solution is prepared and evaporated to dryness by lyophilization; in vial B, vitamin D is dissolved in benzyl alcohol and then diluted in water for injections. The solution is then reconstituted at the time of use, by using the vial B as a solvent of the lyophilized product of vial A and administered every 3 months by intramuscular or subcutaneous injection. For cancer indications vial A can be prepared with 6 mg of ibandronate to be administered every 3-4 weeks, or, for the same purpose, two 3 mg vials can be used, diluted with modified quantities of the solvent of vial B.

### Example 4

### Studies on solubilization and stability of vitamin D in aqueous formulations with benzyl alcohol and bisphosphonates, in comparison to the use of polysorbate or lecithin

To assess the degree of solubilization and stability of vitamin D obtained with the composition formulation according to the present invention, we evaluated the amount of vitamin D in a formulation prepared as in Example 1., in comparison with a formulation prepared as in Example 4. and 15. of the aforementioned patent WO 2008/116809 (which contain an antioxidant, respectively, a-tocopherol and butylated hydroxytoluene), suitably replacing zoledronic acid with 200 mg of clodronic acid. Vitamin D was measured at the end of the preparation, after heat sterilization and after sterilization with sterilizing filters, in which case the comparison was made also in subsequent times. The results obtained are shown in Tables I and II.

**Table I: vitamin D values and presence of particulate material in the formulations under examination (A = formulation with 1% of benzyl alcohol; B = formulation with 0.5% ethanol, 0.1% polysorbate and 0.05% a-tocopherol; C = formulation with 0.5% ethanol, 0.001% butylated hydroxytoluene and 0.1% of soybean lecithin at 70%).**

| **Type of test** | **Post preparation Value** | | | **Post moist heat sterilization Value** | | | **Post sterilization by sterile filtration Value** | | |
|---|---|---|---|---|---|---|---|---|---|
| | A | B | C | A | B | C | A | B | C |
| Vitamin D assessment (% of maximal) | 100 % | 100 % | 100 % | 99.9 % | 92.7 % | 91.6 % | 98.2 % | 95.0 % | 94.4 % |
| Particulate matter ≥ 10 µm per ml | 0 | 5 | 4 | 0 | 12 | 8 | 0 | 8 | 6 |

**Table II: values of vitamin D and the presence of particulate material in the formulations under examination at 6 months after sterilization by sterile filtration under the conditions shown in the table (A = formulation with 1% of benzyl alcohol; B = formulation with 0.5% of ethanol, 0.1% of polysorbate and 0.05% of a-tocopherol; C = formulation with 0.5% ethanol, 0.001% butylated hydroxytoluene and 0.1% of soybean lecithin at 70%).**

| **Type of test** | **Post-preparation values** | | | **Values at 6 months (25 °C)** | | | **Values at 6 months (40 °C/75%)** | | |
|---|---|---|---|---|---|---|---|---|---|
| | A | B | C | A | B | C | A | B | C |
| Vitamin D assessment (% of maximal) | 100% | 100 % | 100 % | 96.3 % | 90.0 % | 89.5 % | 95.3 % | 84.3. % | 85.8% |
| Particulate matter ≥ 10 µm per ml | 0 | 6 | 3 | 0 | 8 | 6 | 0 | 10 | 8 |

### Example 5

### Studies on solubilization and stability of vitamin D in aqueous formulations with benzyl alcohol and bisphosphonates, in comparison to the use of a lipid emulsion

The same tests of the previous study, limited sterilization by sterile filtration, were made in comparison to a preparation with lipid emulsion as the vehicle for vitamin D and bisphosphonates, as described in the patent: WO 2011/063 952. The emulsion was prepared, with the components described in Example 9. of the cited patent, and with procedures that would allow size of lipid particles ≤ 0.1 microns. Results are reported in Table III.

**Table III: vitamin D values and the presence of particulate material in the formulations under examination at 6 months after sterilization by sterile filtration (A = formulation with 1% of benzyl alcohol; D = formulation with 20% of soybean lipids).**

| **Type of test** | **Post-preparation values** | | **Values at 6 months (25 ° C)** | | **Values at 6 months (40 °C/75%)** | |
|---|---|---|---|---|---|---|
| | A | D | A | D | A | D |
| Vitamin D assessment (% of maximal) | 100% | 100% | 99.9% | 94.7% | 99.5% | 92.8% |
| Particulate matter ≥ 10 µm per ml | 0 | 6 | 0 | 8 | 0 | 10 |

### Example 6

### Pharmacokinetic studies on the absorption of vitamin D after intramuscular and subcutaneous administration:

### Example 6a

Pharmacokinetic studies on the absorption of vitamin D after intramuscular administration of a formulation based on clodronate, vitamin D and 1% benzyl alcohol

### Materials and methods

Seventy-five Wistar rats of about 200 g of weight, divided into 3 groups of 25 animals, were used. The 3 groups received intramuscularly, in the body of the right tibialis anterior muscle in a volume of 0.5 ml/kg (0.1 ml/rat), 25 mg/kg of sodium clodronate and 0.125 mg/kg (0.025 mg/rat) of a mixture of vitamin D3 (cholecalciferol) and [1,2-3H] cholecalciferol (10% by weight; 0:51 Ci/mmole), respectively diluted in an aqueous solution containing 10mg/ml of benzyl alcohol (formulation A), or 0.5% ethanol, 0.2% polysorbate and 0.05% of a-tocopherol (formulation B) or in a 20% lipid emulsion, corresponding to the example 9 of the patent application WO 2011/063952 (formulation D). Five rats of each group were sacrificed after 6 and 24 hours and after 1, 2 and 3 weeks following administration, for the determination of plasma concentrations of 25-hydroxy-cholecalciferol (calcidiol), formed by metabolism.

Data of vitamin D absorption after intramuscular administration with the 3 different formulations are shown in TABLE IV.

**TABLE IV. Plasma concentrations of calcidiol after intramuscular administration of vitamin D3 (10000 UI/ml-1000UI/rat) in the test formulations described above**

| Group | 6 hours ng/ml | at 24 hours ng/ml | 1 weeks ng/ml | 2 weeks ng/ml | 3 weeks ng/ml |
|---|---|---|---|---|---|
| Formulation A | 28.2 +/-19.5 | 37.5 +/-12.8 | 51.5 +/-14.3 | 55.9 +/-17.5 | 42.4 +/-16.7 |
| Formulation B | 8.4 +/-6.5 | 9.2 +/-5.2 | 13.3 +/-4.1 | 26.5 +/-11.1 | 33.2 +/-16.9 |
| Formulation C | 17.4 +/-15.1 | 26.3 +/-13.2 | 42.5 +/-11.2 | 48.6 +/-13.5 | 51.5 +/-14.4 |

The formulation containing benzyl alcohol has achieved, already after 6 hours and until the second week, plasma levels of calcidiol higher than those obtained with the formulations of the comparison, i.e. containing 0.5% of ethanol, 0.2% of polysorbate and 0.05% of a-tocopherol or 20% of soy lipids.

### Example 6b

### Pharmacokinetic study on the absorption of vitamin D after subcutaneous administration of sodium neridronate in rats.

The test was performed with methods and formulations similar to those used for the test of Example 6a (replacing clodronate with suitable quantities of ibandronic acid), obtaining similar results, demonstrating that the properties of the formulation we found are expressed also for this route of administration.

### Example 7

### Studies on water solubilization of bisphosphonates and vitamin D with benzyl alcohol and other organic solvents

In order to assess the degree of water solubilization of bisphosphonates and vitamin D by addition of variable amounts of benzyl alcohol and other water mixable organic solvents, 2 mother solutions at concentration of 100 mg disodium clodronate or 5 mg zoledronic acid in 2 ml water for injection, examples of respectively high and low dose bisphosphonates, were prepared and buffered with sodium bicarbonate to pH 6.5.

Two-ml samples of both solutions with the two bisphosphonates mentioned above were then diluted with water to approx. 3 ml (3 samples: more precisely 2997 µl, 2991µl, and 2970 µl), to 2.7 ml (1 sample) and to 2.4 ml (1 sample).A mother solution containing cholecalciferol in benzyl alcohol or other water mixable organic solvents in a ratio of 0.35 mg : 3.0 mg respectively (11.67% w/w) was also prepared and 2 series of 5 samples of 3.35 mg of it were taken and kept as such (1 sample) or diluted with 6.0 mg, 27.0 mg, 297.0 and 597.0 mg respectively of the same solvent (4 samples).The 5 cholecalciferol solution samples were then added, under slow stirring, to the 5 solution samples of each bisphosphonate in the order, so as to give mixtures with solvent concentration of 0.1, 0.3, 1.0, 10.0 and 20.0% (w/v), left to stand for 10 minutes and then examined according to the microscopic particle count test or visually (an instrumental particle counter was unnecessary).

The results are reported in Table Va and Vb.

**Table Va: particulate matter in water solutions of disodium clodronate 100 mg/3ml and cholecalciferol 0.35mg / 3ml, containing different % concentrations (w/v) of organic solvents.**

| Type of test | Particulate matter ≥ 10µm per ml | | | | |
|---|---|---|---|---|---|
| % Solvent | 0.1 | 0.3 | 1.0 | 10 | 20 |
| Benzyl alcohol | 8 | 0 | 0 | 0 | * |
| Ethanol | 75 | 23 | 48 | ** | *** |
| Polyethylene glycol 400 (PEG 400) | 40 | 18 | 13 | * | ** |
| Polyethylene glycol octadecyl ether (Brij® 76) | 92 | 48 | 66 | ** | *** |
| Polyoxyethylene 8 stearate (Myrj®45) | 64 | 14 | 18 | ** | *** |
| Polyethylene glycol 40 monoricinoleate | 35 | 10 | 8 | ** | ** |
| Polyoxyethylene sorbitane monopalmitate (Tween ® 40) | 42 | 12 | 8 | * | ** |
| Sorbitane monoleate (SPAN® 80) | 83 | 74 | 56 | ** | *** |
| Cremophor EL | 22 | 13 | 7 | * | ** |

| | | | | | |
|---|---|---|---|---|---|
| *opalescent; **turbid; ***precipitate Note: the number of particles ≥ 25µm was in the range of 10-30% of the total and is not reported. | | | | | |

**Table Vb: particulate matter in water solutions of zoledronic acid 5.0 mg/3ml and cholecalciferol 0.35 mg / 3ml, containing different % concentrations (w/v) of organic solvents.**

| Type of test | Particulate matter ≥ 10µm per ml | | | | |
|---|---|---|---|---|---|
| % Solvent | 0.1 | 0.3 | 1.0 | 10.0 | 20.0 |
| Benzyl alcohol | 10 | 0 | 0 | 0 | * |
| Ethanol | 68 | 22 | 16 | * | ** |
| Polyethylene glycol 400 (PEG 400) | 42 | 18 | 9 | * | ** |
| Polyethylene glycol 40 monoricinoleate | 25 | 12 | 7 | * | ** |
| Polyoxyethylene sorbitane monopalmitate (Tween ® 40) | 18 | 14 | 13 | * | * |
| Cremophor EL | 31 | 15 | 8 | * | * |

| | | | | | |
|---|---|---|---|---|---|
| *opalescent; **turbid; ***precipitate Note: the number of particles ≥ 25µm was in the range of 10-25% of the total and is not reported. | | | | | |

It results from the above data that solutions of bisphosphonates and cholecalciferol containing benzyl alcohol in the range of 0.3-10.0% (w/v) have the unique property of giving limpid solutions free from particulate matter deriving from one or both active ingredients, in comparison to solutions containing a series of other known solvents or surfactants.

## Claims

1. A composition for pharmaceutical use comprising a compound of the class of bisphosphonates and a vitamin of the D group selected from the group consisting of vitamin D3 (cholecalciferol) and vitamin D2 (ergocalciferol), in which all the components are dissolved in an aqueous solution containing benzyl alcohol at a concentration of between 0.3% (3 mg/ml) and 10% (100 mg/ml) w/v and in a suitable volume for intramuscular and subcutaneous administration.

2. The composition according to claim 1, wherein the content of the compound of the class of bisphosphonates is between 1 mg and 400 mg, preferably between 3 mg and 200 mg.

3. The composition according to any one of the preceding claims, wherein the vitamin of the D group is vitamin D3 and its content is not less than 2700 IU, preferably between 2700 IU and 900000 IU, more preferably between 5600 IU and 90000 IU.

4. The composition according to any one of claims 1 and 2, wherein the vitamin of the D group is vitamin D3 and the concentration of vitamin D3 is not less than 540 IU / ml, and always between 540 IU / ml and 1.8 million IU / ml.

5. The composition according to any one of claims 1 and 2, wherein the vitamin of the D group is vitamin D2 (ergocalciferol) and is in such a content as to provide a biological activity equivalent to the one provided by the vitamin D3 contents specified in claim 3.

6. The composition according to any one of claims 1 and 2, wherein the vitamin of the D group is vitamin D2 (ergocalciferol) and is in such a concentration as to provide a biological activity equivalent to the one provided by the vitamin D3 concentrations specified in claim 4.

7. The composition according to any one of the preceding claims, wherein the concentration of benzyl alcohol is between 0.5% (5mg/ml) and 5% (50 mg/ml) and preferably between 1% (10 mg/ml) and 3% (30 mg/ml).

8. The composition according to any one of the preceding claims, in which the volume is not more than 5 ml, preferably between 0.5 and 5.0 ml, more preferably between 0.5 and 3.5 ml, and even more preferably between 1 and 2.0 ml.

9. The composition according to any one of the preceding claims, in which the compound of the class of bisphosphonates is selected from the group comprising clodronate, alendronate, etidronate, neridronate, pamidronate, risedronate, zoledronate, ibandronate, incadronate, olpadronate, tiludronate, alone, or a combination thereof.

10. The composition according to any one of the preceding claims, in which the compound of the class of bisphosphonates includes the acid form, or salts thereof with pharmaceutically acceptable bases,usually sodium salts at various degrees of salification, as well as any hydrated and anhydrous forms, any racemates, enantiomers or diastereoisomers and any amorphous and crystalline polymorphous forms.

11. A pharmaceutical formulation for intramuscular and subcutaneous administration, comprising a composition according to any one of the preceding claims and at least one pharmaceutically acceptable excipient and / or adjuvant.

12. A pharmaceutical formulation according to claim 11, wherein said at least one excipient and / or adjuvant is selected from the group of sugars such as glucose, sucrose, lactose or mannose; from the group of polyalcohols, such as glycerol, xylitol and others; of preservatives such as parabens and others; of ionic and nonionic surfactants, such as sodium deoxycholate, sodium glycocholate and other bile salts, sodium lauryl sulphate, Tweens and Cremophor; of basic, neutral or acidic buffers, such as alkali carbonates or bicarbonates, phosphates or TRIS buffer, or in general from the group of water-miscible and pharmaceutically acceptable organic solvents, in limited amounts; of antioxidants such as tocopherol, ascorbyl palmitate, butyl-hydroxyanisole and butyl-hydroxytoluene; stabilizers and co-solvents; or the formulation is suitably protected in an inert atmosphere such as nitrogen.

13. The pharmaceutical formulation according to claim 12, containing a local anesthetic, preferably lidocaine.

14. A method for producing the pharmaceutical formulation according to any one of claims 11 to 13, which comprises dissolving the compound of the class of bisphosphonates and the vitamin of the D group in an aqueous solution with benzyl alcohol together with said at least one pharmaceutically acceptable excipient and / or adjuvant and bringing the obtained solution into a sterile condition ready for administration.

15. A method of producing the pharmaceutical formulation according to any one of claims 11 to 13, which comprises:
a) dissolving the compound of the class of bisphosphonates in an aqueous solution, optionally together with said at least one pharmaceutically acceptable excipient and / or adjuvant,
b) dissolving the vitamin of the D group in an aqueous solution containing benzyl alcohol, optionally together with said at least one pharmaceutically acceptable excipient and / or adjuvant, thus obtaining two separate solutions, which are suitable for beingeither combined extemporaneously before administration or injected separately in a sequential manner.

16. A method for producing the pharmaceutical formulation according to any one of claims 11 to 13, comprising the steps of
- providing a first component consisting of the compound of the class of bisphosphonates and said at least one pharmaceutically acceptable excipient and/or adjuvant
in the dry state, as a lyophilized substance or as a sterile powder;
- providing a second component consisting of benzyl alcohol and vitamin D dissolved in water, wherein the two components are preferably contained in an ampoule-syringe and are adapted to be combined extemporaneously prior to injection.

17. A pharmaceutical formulation for intramuscular and subcutaneous administration according to claims 11-13 for use in the prevention and/or treatment of all bone diseases sensitive to the action of the bisphosphonate and of vitamin D, such as loss of bone tissue and/or its increased turnover, and in the states of vitamin D deficiency.

18. A pharmaceutical formulation for intramuscular and subcutaneous administration according to claims 11-13 for use in the prevention and/or treatment of osteoporosis of any origin, such as postmenopausal osteoporosis, steroid or glucocorticoid induced osteoporosis, male osteoporosis, osteoporosis induced by other diseases or idiopathic; Paget's disease; osteoarthritis; localized bone loss associated with bones implants or with osteolysis; bone fractures; bone diseases related to bone metastases; incomplete or imperfect ossification; periodontal disease and tooth loss; malignant hypercalcemia; multiple myeloma; osteopenia, including osteopenia induced by prolonged immobilization and by bone metastases.

## Patentansprüche

1. Zusammensetzung für die pharmazeutische Verwendung, die eine Verbindung der Klasse von Bisphosphonaten und ein Vitamin der D-Gruppe umfasst, ausgewählt aus der Gruppe bestehend aus Vitamin D3 (Cholecalciferol) und Vitamin D2 (Ergocalciferol), wobei alle der Komponenten in einer wässrigen Lösung gelöst sind, die Benzylalkohol in einer Konzentration zwischen 0,3 % (3 mg/ml) und 10 % (100 mg/ml) w/v und in einem geeigneten Volumen für die intramuskuläre und subkutane Verabreichung enthält.

2. Zusammensetzung nach Anspruch 1, wobei der Gehalt der Verbindung der Klasse von Bisphosphonaten zwischen 1 mg und 400 mg, bevorzugt zwischen 3 mg und 200 mg liegt.

3. Zusammensetzung nach einem der vorhergehenden Ansprüche, wobei das Vitamin der D-Gruppe Vitamin D3 ist und sein Gehalt nicht weniger als 2700 IE ist, bevorzugt zwischen 2700 IE und 900.000 IE, noch bevorzugter zwischen 5600 IE und 90.000 IE liegt.

4. Zusammensetzung nach einem der Ansprüche 1 und 2, wobei das Vitamin der D-Gruppe Vitamin D3 ist und die Konzentration des Vitamins D3 nicht weniger als 540 IE/ml beträgt und immer zwischen 540 IE/ml und 1,8 Millionen IE/ml liegt.

5. Zusammensetzung nach einem der Ansprüche 1 und 2, wobei das Vitamin der D-Gruppe Vitamin D2 (Ergocalciferol) ist und in einem derartigen Gehalt vorliegt, dass es eine biologische Aktivität äquivalent derjenigen bereitstellt, die durch die in Anspruch 3 angegebenen Vitamin D3-Gehalte bereitgestellt wird.

6. Zusammensetzung nach einem der Ansprüche 1 und 2, wobei das Vitamin der D-Gruppe Vitamin D2 (Ergocalciferol) ist und in einer derartigen Konzentration vorliegt, dass es eine biologische Aktivität äquivalent derjenigen bereitstellt, die durch die in Anspruch 4 angegebenen Vitamin D3-Konzentrationen bereitgestellt wird.

7. Zusammensetzung nach einem der vorhergehenden Ansprüche, wobei die Konzentration von Benzylalkohol zwischen 0,5 % (5 mg/ml) und 5 % (50 mg/ml) und bevorzugt zwischen 1 % (10 mg/ml) und 3 % (30 mg/ml) liegt.

8. Zusammensetzung nach einem der vorhergehenden Ansprüche, wobei das Volumen nicht mehr als 5 ml beträgt, bevorzugt zwischen 0,5 und 5,0 ml, noch bevorzugter zwischen 0,5 und 3,5 ml und sogar noch bevorzugter zwischen 1 und 2,0 ml liegt.

9. Zusammensetzung nach einem der vorhergehenden Ansprüche, wobei die Verbindung der Klasse von Bisphosphonaten aus der Gruppe ausgewählt wird, welche Clodronat, Alendronat, Etidronat, Neridronat, Pamidronat, Risedronat, Zoledronat, Ibandronat, Incadronat, Olpadronat, Tiludronat, als solchem oder in einer Kombination davon, umfasst.

10. Zusammensetzung nach irgendeinem der vorhergehenden Ansprüche, wobei die Verbindung der Klasse von Bisphosphonaten die saure Form oder Salze davon mit pharmazeutisch akzeptablen Basen, gewöhnlich Natriumsalze in verschiedenen Salzbildungsgraden sowie beliebigen hydratisierten und wasserfreien Formen, beliebigen Racematen, Enantiomere oder Diastereomere und beliebigen amorphen und kristallinen polymorphen Formen beinhaltet.

11. Pharmazeutische Formulierung für die intramuskuläre und subkutane Verabreichung umfassend eine Zusammensetzung nach einem der vorhergehenden Ansprüche und mindestens einen pharmazeutisch akzeptablen Träger und/oder Hilfsmittel.

12. Pharmazeutische Formulierung nach Anspruch 11, wobei mindestens ein Träger und/oder Hilfsmittel ausgewählt ist aus der Gruppe von Zuckern wie Glucose, Saccharose, Lactose oder Mannose; aus der Gruppe von Polyalkoholen wie Glycerin, Xylit und anderen; von Konservierungsmitteln wie Parabenen und anderen; von ionischen und nichtionischen Tensiden wie Natriumdeoxycholat, Natriumglycocholat und anderen Gallensalzen, Natriumlaurylsulfat, Tweens und Cremophor; von basischen, neutralen oder sauren Puffern wie Alkalicarbonaten oder -bicarbonaten, Phosphaten oder TRIS-Puffer oder im Allgemeinen aus der Gruppe von mit Wasser mischbaren und pharmazeutisch akzeptablen organischen Lösungsmitteln in begrenzten Mengen; von Antioxidationsmitteln wie Tocopherol, Ascorbylpamitat, Butylhydroxyanisol und ,Butyl'-hydroxytoluol; Stabilisatoren und Hilfslösungsmitteln; oder die Formulierung ist auf geeignete Weise in einer inerten Atmosphäre wie Stickstoff geschützt.

13. Pharmazeutische Formulierung nach Anspruch 12, die ein Lokalanästhetikum, bevorzugt Lidocain enthält.

14. Verfahren für die Herstellung der pharmazeutischen Formulierung nach einem der Ansprüche 11 bis 13, das das Lösen der Verbindung der Klasse von Bisphosphonaten und des Vitamins der D-Gruppe in einer wässrigen Lösung mit Benzylalkohol zusammen mit dem mindestens einen pharmazeutisch akzeptablen Träger und/oder Hilfsmittel und das Einbringen der erhaltenen Lösung in einen sterilen Zustand, der für die Verabreichung bereit ist, umfasst.

15. Verfahren für die Herstellung der pharmazeutischen Formulierung nach einem der Ansprüche 11 bis 13, das umfasst:
a) Lösen der Verbindung der Klasse von Bisphosphonaten in einer wässrigen Lösung, wahlweise zusammen mit dem mindestens einen pharmazeutisch akzeptablen Träger und/oder Hilfsmittel,
b) Lösen des Vitamins der D-Gruppe in einer wässrigen Lösung, die Benzylalkohol enthält, wahlweise zusammen mit dem mindestens einen pharmazeutisch akzeptablen Träger und/oder Hilfsmittel, wodurch zwei einzelne Lösungen erhalten werden, die geeignet sind, entweder improvisiert vor der Verabreichung kombiniert oder getrennt auf sequenzielle Weise injiziert zu werden.

16. Verfahren für die Herstellung der pharmazeutischen Formulierung nach irgendeinem der Ansprüche 11 bis 13, umfassend die Schritte des
- Bereitstellens einer ersten Komponente, die aus der Verbindung der Klasse von Bisphosphonaten und dem mindestens einen pharmazeutisch akzeptablen Träger und/oder Hilfsmittel besteht
im trockenen Zustand als lyophilisierte Substanz oder als steriles Pulver;
- Bereitstellens einer zweiten Komponente, die aus Benzylalkohol und in Wasser gelöstem Vitamin D besteht, wobei die beiden Komponenten bevorzugt in einer Ampullenspritze enthalten sind und geeignet sind, vor der Injektion improvisiert kombiniert zu werden.

17. Pharmazeutische Formulierung zur intramuskulären und subkutanen Verabreichung nach den Ansprüchen 11 - 13 zur Verwendung in der Prävention und/oder Behandlung aller Knochenkrankheiten, die auf die Wirkung des Bisphosphonats und von Vitamin D ansprechen, wie beispielsweise Verlust von Knochengewebe und/oder seinem erhöhten Umsatz und in den Zuständen von Vitamin D-Mangel.

18. Pharmazeutische Formulierung für die intramuskuläre und subkutane Verabreichung nach den Ansprüchen 11 - 13 zur Verwendung in der Prävention und/oder Behandlung von Osteoporose irgendeines Ursprung, wie postmenopausaler Osteoporose, durch Steroid oder Glucocorticoid induzierter Osteoporose, männlicher Osteoporose, Osteoporose, die durch andere Krankheiten induziert oder idiopathisch ist; Morbus Paget; Osteoarthritis; lokalisiertem Knochenverlust, der mit Knochenimplantaten oder mit Osteolyse verbunden ist; Knochenbrüchen, Knochenkrankheiten, die mit Knochenmetastasen verbunden sind; vollständiger oder mangelhafter Ossifikation; paradontaler Krankheit und Zahnverlust; bösartiger Hyperkalzämie; multiplem Myelom; Osteopenie, einschließlich durch längere Immobilisierung und Knochenmetastasen induzierte Osteopenie.

## Revendications

1. Composition pour un usage pharmaceutique, comprenant un composant de la classe des bisphosphonates et une vitamine du groupe D choisie dans le groupe comprenant la vitamine D3 (cholecalciférol) et la vitamine D2 (ergocalciférol), dans laquelle tous les composants sont dissous dans une solution aqueuse contenant de l'alcool benzylique à une concentration comprise entre à 0,3 % (3 mg/ml) et 10 % (100 mg/ml) p/v et dans un volume approprié pour une administration par voie intramusculaire et sous-cutanée.

2. Composition selon la revendication 1, dans laquelle la teneur du composant de la classe des bisphosphonates est comprise entre 1 mg et 400 mg, de préférence entre 3 mg et 200 mg.

3. Composition selon l'une quelconque des revendications précédentes, dans laquelle la vitamine du groupe D est la vitamine D3 et sa teneur n'est pas inférieure à 2 700 Ul, de préférence comprise entre 2 700 Ul et 900 000 Ul, de façon privilégiée comprise entre 5 600 Ul et 90 000 Ul.

4. Composition selon l'une quelconque des revendications 1 et 2, dans laquelle la vitamine du groupe D est la vitamine D3 et la concentration en vitamine D3 n'est pas inférieure à 540 Ul/ml, et toujours comprise entre 540 Ul/ml et 1,8 million Ul/ml.

5. Composition selon l'une quelconque des revendications 1 et 2, dans laquelle la vitamine du groupe D est la vitamine D2 (ergocalciférol) et elle se trouve à une concentration telle qu'elle fournit une activité biologique équivalente à celle fournie par les teneurs en vitamine D3 spécifiées dans la revendication 3.

6. Composition selon l'une quelconque des revendications 1 et 2, dans laquelle la vitamine du groupe D est la vitamine D2 (ergocalciférol) et elle se trouve à une concentration telle qu'elle fournit une activité biologique équivalente à celle fournie par les concentrations en vitamine D3 spécifiées dans la revendication 4.

7. Composition selon l'une quelconque des revendications précédentes, dans laquelle la concentration en alcool benzylique est comprise entre 0,5 % (5 mg/ml) et 5 % (50 mg/ml), de préférence entre 1 % (10 mg/ml) et 3 % (30 mg/ml).

8. Composition selon l'une quelconque des revendications précédentes, dans laquelle le volume n'est pas supérieur à 5 ml, de préférence compris entre 0,5 et 5,0 ml, plus préférablement entre 0,5 et 3,5 ml, et de façon privilégiée entre 1 et 2,0 ml.

9. Composition selon l'une quelconque des revendications précédentes, dans laquelle le composant de la classe des bisphosphonates est choisi dans le groupe comprenant le clodronate, l'alendronate, l'étidronate, le néridronate, le pamidronate, le risédronate, le zolédronate, l'ibandronate, l'incadronate, l'olpadronate, le tiludronate, pris seuls, ou une combinaison de ceux-ci.

10. Composition selon l'une quelconque des revendications précédentes, dans laquelle le composant de la classe des bisphosphonates inclut la forme acide, ou des sels de celle-ci avec des bases acceptables du point de vue pharmaceutique, habituellement des sels de sodium à des degrés divers de salification, ainsi que toutes les formes hydratées et anhydres, tous les racémates, les énantiomères ou diastéréoisomères et toutes les formes amorphes et polymorphes cristallines.

11. Formulation pharmaceutique pour administration par voie intramusculaire ou sous-cutanée, comprenant une composition selon l'une quelconque des revendications précédentes et au moins un excipient et/ou adjuvant acceptable du point de vue pharmaceutique.

12. Formulation pharmaceutique selon la revendication 11, dans laquelle ledit au moins un excipient et/ou adjuvant est choisi dans le groupe des sucres tels que le glucose, le saccharose, le lactose ou le mannose ; dans le groupe des polyalcools tels que le glycérol, le xylitol et autres ; des agents conservateurs tels que les parabènes et autres ; des agents tensio-actifs ioniques et non ioniques, tels que le désoxycholate de sodium, le glycocholate de sodium et autres sels biliaires, le laurylsulfate de sodium, Tweens et Cremophor ; des tampons basiques, neutres ou acides, tels qu'un tampon de carbonates ou de bicarbonates alcalins, de phosphates ou un tampon TRIS, ou de façon générale du groupe des solvants organiques miscibles à l'eau et acceptables du point de vue pharmaceutique, en quantités limitées ; des antioxydants tels que le tocophérol, le palmitate d'ascorbyle, l'hydroxyanisole butylé et l'hydroxytoluène butylé ; des stabilisants et des co-solvants ; ou la formulation est protégée de manière appropriée dans une atmosphère inerte telle que l'azote.

13. Formulation pharmaceutique selon la revendication 12, contenant un anesthésique local, de préférence de la lidocaïne.

14. Procédé de production de la formulation pharmaceutique selon l'une quelconque des revendications 11 à 13, lequel procédé comprend la dissolution du composant de la classe des bisphosphonates et de la vitamine du groupe D dans une solution aqueuse avec de l'alcool benzylique conjointement avec ledit au moins un excipient et/ou adjuvant acceptable du point de vue pharmaceutique, et mise de la solution obtenue sous une forme stérile prête à être administrée.

15. Procédé de production de la formulation pharmaceutique selon l'une quelconque des revendications 11 à 13, lequel comprend :
a) la dissolution du composant de la classe des bisphosphonates dans une solution aqueuse, de façon optionnelle conjointement avec ledit au moins un excipient et/ou adjuvant acceptable du point de vue pharmaceutique,
b) la dissolution de la vitamine du groupe D dans une solution aqueuse contenant de l'alcool benzylique, de façon optionnelle conjointement avec ledit au moins un excipient et/ou adjuvant acceptable du point de vue pharmaceutique, en obtenant ainsi deux solutions séparées qui sont aptes à être soit combinées extemporanément avant administration, soit injectées séparément de façon séquentielle.

16. Procédé de production de la formulation pharmaceutique selon l'une quelconque des revendications 11 à 13, lequel comprend les étapes consistant à :
- préparer un premier composant consistant en le composant de la classe des bisphosphonates et dudit au moins un excipient et/ou adjuvant acceptable du point de vue pharmaceutique
à l'état sec, sous forme d'une substance lyophilisée ou d'une poudre stérile ;
- préparer un deuxième composant consistant en alcool benzylique et la vitamine D dissouts dans l'eau, les deux composants étant de préférence contenus dans une seringue-ampoule et étant adaptés à être combinés extemporanément avant l'injection.

17. Formulation pharmaceutique pour une administration par voie intramusculaire ou sous-cutanée selon l'une des revendications 11 à 13 pour une utilisation dans la prévention et/ou le traitement de maladies osseuses sensibles à l'action du bisphosphonate et de la vitamine D, telles que la perte de tissus osseux et/ou leur renouvellement accru, et dans les situations de manque de vitamine D.

18. Formulation pharmaceutique pour une administration par voie intramusculaire ou sous-cutanée selon l'une des revendications 11 à 13 pour une utilisation dans la prévention et/ou le traitement de l'ostéoporose de toute origine, telle que l'ostéoporose post-ménopause, l'ostéoporose induite par stéroïdes ou glucocorticoïdes, l'ostéoporose masculine, l'ostéoporose induite par d'autres maladies ou idiopathie ; la maladie de Paget ; l'ostéo-arthrite ; la perte osseuse localisée associée à des implants osseux ou à de l'ostéolyse ; des fractures osseuses ; des maladies osseuses en relation avec des métastases osseuses ; une ossification incomplète ou imparfaite ; des maladies périodontales et la perte de dent ; l'hypercalcémie maligne ; le myélome multiple ; l'ostéopénie, y compris l'ostéopénie induite par une immobilisation prolongée et par des métastases osseuses.
